# EUROPEAN PATENT APPLICATION

(11) **EP 2 363 126 A1**
(43) Date of publication of application: **07.09.2011**
(21) Application number: 10155501.9
(22) Date of filing: 04.03.2010
(51) Int. Cl.: A61K 31/205, A61K 31/7036, A61P 27/02

(54) **Composition comprising as active ingredient L-carnitine in combination with hydroxykynurenine-O-beta-DL-glucoside, for the prevention and/or treatment of pathologies of the eye due to ultraviolet radiation**

(71) Applicant: SIGMA-TAU Industrie Farmaceutiche Riunite S.p.A., 00144 Roma (IT)
(72) Inventor: Pescosolido, Nicola, 00181, Rome (IT); Koverech, Aleardo, 00165, Rome (IT)

(57) **Abstract**

It is described a composition in the form of eye drops or ointment, comprising as active ingredients L-carnitine or a physiologically acceptable salt thereof; and 3-Hydroxykynurenine O-β -DL-glucoside, useful for preventing or treating pathologies of the eyes due to ultraviolet radiation.

## Description

The present invention relates to a physiological supplement or medicament in the form eye drops or ointment, comprising as active ingredients L-carnitine, in combination with a solar filter, for the prevention or treatment of pathologies of the eye due to ultraviolet radiation.

The solar filter according to the present invention is 3-Hydroxykynurenine (3-HKG) or derivatives thereof, or mixtures thereof. The composition of the invention may further comprising taurine, glycerol, resveratrol, sodium jaluronate, vitamins and microelements which increase the activity of the combination of the invention.

Diseases of the eye due to ultraviolet radiation include age-related cataract, non age-related cataract; pterygium; Macular Degeneration such as age related maculopathy (ARM), non age related maculopathy (nARM), age related macular degeneration (AMD); attinic photokeratitis and conjunctivitis.

Although ultra-violet radiation has long been recognized as a factor in the development of cutaneous cancer, aging of the skin, and mutagenic changes, it is only within the last decade or less that ultra-violet radiation has been universally recognized as a causative factor in ocular pathogenesis.

In humans, the eye has evolved into a sophisticated organ having neurophysiologic responses to photons in a certain portion of the electromagnetic spectrum, that provides a constant detailed map of the immediate environment. The action spectrum for these responses lie primarily within the 400-700 NM wavelength range, which has been labelled the visible spectrum or "Light".

Because solar UV radiation is present during most of the daylight hours, the eye may be exposed daily to some amount of solar ultraviolet radiation throughout life.

However, unlike the skin, the ocular system does not develop tolerance to repeated ultraviolet exposure. Swelling or shrinking of groups of corneal epithelial cells leads to visibly recognizable stippling or irregular mosaic granulation of the corneal surface. With UV doses greater than the threshold for photokeratitis, surface epithelial cells show nuclear fragmentation, mid-epithelial cells show vacuole formation, and basel cells show inhibition of mitosis and clouding of the corneal stroma occurs. Inflammation is also present in the conjunctiva where vasodilatation, oedema, and inflammatory cell infiltrate is followed by desquamation.

When the cornea is damaged or replaced by surgery, the eye in general becomes very sensitive and the retina becomes vulnerable to ultra violet radiation. Also the retina of diabetic patients is particularly vulnerable to ultra violet radiation.

This is the opposite of what happens with a healthy cornea. In the healthy or undamaged eye, absorption of radiation by the cornea and lens of the human eye is such that very little radiation of wavelengths shorter than 390 NM reaches the retina.

The retina (tunica interna) is a delicate nervous membrane, upon which the images of external objects are received. Its outer surface is in contact with the choroid; its inner with the vitreous body. Behind, it is continuous with the optic nerve; it gradually diminishes in thickness from behind forward and extends nearly as far as the ciliary body, where it ends in a jagged margin, the ora serrata. Here the nervous tissues of the retina end, but a thin prolongation of the membrane extends forward over the back of the ciliary processes and iris, forming the pars ciliatis retinae and pars inidica retinae. This forward prolongation consists of the pigmentary layer of the retina together with a stratum of columnar epithelium. The retina is soft, semitransparent, and of a purple tint in the fresh state, owing to the presence of a colouring material named rhodopsin or visual purple; however, it soon becomes clouded, opaque, and bleached when exposed to sunlight.

Cataract is a major cause of visual impairment and blindness worldwide. Cataracts are a cloudiness of the lens inside the eye which occurs over a period of many years. Laboratory studies have implicated UV radiation as a causal factor for cataract. Furthermore, epidemiological studies have shown that cataract is associated with a history of higher exposure to UV-radiation (Doc Ophthalmol. 1994-1995;88(3-4):339).

Pterygium is a growth of tissue on the white of the eye that may extend onto the clear cornea where it can block vision. It is seen most commonly in people who work outdoors in the sun and wind, and its prevalence is related to the amount of UV exposure. It can be removed surgically, but often recurs, and can cause cosmetic concerns and visual loss if untreated.

The acute exposure to UV-radiation cause conjunctivitis and corneal inflammation reaction known as attinic photokeratitis. The clinical progress or picture of photokeratitis follows a characteristic course. For example, after exposure, there is a period of latency which varies somewhat inversely with the amount of exposure. The latent period may be as short as 30 minutes or as long as 24 hours but it is typically 6 to 12 hours.

Conjunctivitis, which is often accompanied by an erythema of the skin surrounding the eyelids, is associated with the sensation of a foreign body or "sand" in the eyes, varying degrees of photophobia (intolerance to light), lacrimation (tearing), and blepharospasm (spasm of lid muscles). Corneal pain can be very severe, and the individual is usually incapacitated for some period of time. These acute symptoms usually last from 6 to 24 hours and almost all discomfort disappears within 48 hours. Very rarely does conjunctivitis causing exposure result in permanent damage.

UV radiation is a contributory cause of macular degeneration.

Macular degeneration, which is the major cause of irreversible vision loss in old people in the United States, is the progressive deterioration of a critical region of the retina called the macula. The macula is a 3-5 mm area in the retina that is responsible for central vision. This disorder leads to irreversible loss of central vision, although peripheral vision is retained. In the early stages, vision may be gray, hazy, or distorted.

Previous uses of carnitines, 3HKG or 7-dehydrocholesterol in the ophthalmological field are already known.

US 5,883,127 relates to the use of acetyl L-carnitine to produce a medicament suitable for the therapeutic treatment of retinopathies, e.g. the age-related maculopathy (ARM) and non-age-related maculopathy (nARM), in which acetyl L-carnitine is administered enterallyl (particularly orally) or parenterally (particularly intramuscularly or intravenously).

U.S. Pat. No. 5,037,851 describes the use of acetyl L-carnitine and its pharmacologically acceptable salts in the therapeutic treatment of cataract.

U.S. Pat. Nos. 5,145,871 and 5,432,199 describe the use of acetyl D-carnitine and its pharmacologically acceptable salts in the therapeutic treatment of glaucoma.

US 5,876,709 relates to the use of 7-dehydrocholesterol for preventing damages of the eyes due to UV-radiation.

US 2004/0042072-A relates to the inclusion of 3-hydroxykynurenine into artificial ocular lens useful for preventing damages due to UV-radiations.

WO2008/077712 relates to the use of an ophthalmic combination compositions comprising as active ingredients L-carnitine or acetyl L-carnitine in a dose of 5% (Formulation 3) or 20% (Formulation 4) respectively and 3-HKG in a dose of 0.1 mg/ml. Said ophthalmic combinations are in a gel form and are endowed with several drawbacks.

In fact an amount of 0.1 mg/mL of 3-HKG:
- is not (completely) soluble in water;
- is not miscible with the others components of the combination; and
- give rise to an ophthalmic composition (eye drops) having a dark colour.

It is evident to any Expert in ophthalmology that an eye drops solution cannot contains solid granules. It is also evident that a dark eye drops solution cannot permit to the patient to see if microorganism are growing, or solid granules are present, into said solution.

None of the publications cited above mention or suggest the use the combination composition, in the form of eye drops, of the present invention.

L-carnitine is used in the cardiovascular field for the treatment of acute and chronic myocardial ischaemia, angina pectoris, heart failure and cardiac arrhythmias. In the nephrological field, L-carnitine is administered to uraemic patients on regular haemodialysis treatment to combat muscular asthenia and the onset of muscle cramps. Other therapeutic uses of L-carnitine relates to the restoration of a normal HDL/LDL + VLDL ratio and total parenteral nutrition.

3-Hydroxykynurenine O-β-D-glucoside is a low molecular weight compound present in the lenses of humans and other primates which acts as intraocular filters by absorbing UV light in the 300-400 nm region (J. Physiol. 1969; 203, 411-417) end prevents UV-induced photodamage to the retina (1988) Exp. Eye Res. 47, 819-824.

Taurine (or 2-aminoethanesulphonic acid) is considered an amino acid, even though it does not possess the characteristic carboxyl group (COOH) but the SO₃H group. Taurine is only present in the animal realm, whereas vegetable foods do not possess this amino acid.

Glycerol is an organic compound, also called glycerin or glycerine. It is a colorless, odorless, viscous liquid that is widely used in pharmaceutical or ophthalmic formulations. Glycerol has three hydrophilic hydroxyl groups that are responsible for its solubility in water and its hygroscopic nature. The glycerol substructure is a central component of many lipids. Glycerol is sweet-tasting and of low toxicity.

Resveratrol is a natural polyphenol present in grapes and wine. Numerous groups of researchers following epidemiological studies which have demonstrated that populations which consume moderate amounts of wine in their habitual diet present a much lower percentage of cardiovascular accidents than do populations whose dietary habits do not include the consumption of wine.

Resveratrol is to be found in grapes and in particular in grape skins, seeds, stalks and vine leaves, particularly of Vitis vinifera, Vitis rotundifolia and Vitis labrusca and, obviously, in the wines obtained from their grapes and in the extracts and powders of the above-mentioned natural products. Resveratrol is also present in the roots of a number of species of the Polygonum genus (Polygonaceae family), such as Polygonum cuspidatum and Polygonum multiflorum.

For the purposes of the present invention, what is meant by "resveratrol" is both trans-3,4'-5-trihydroxystilbene (i.e. resveratrol proper) and the cis isomer, the respective glycosides and extracts and powders containing resveratrol obtained from any suitable plant species.

Sodium hyaluronate is a very well known compound used to protect corneal epithelial cells, especially in patients with dry eye syndrome or with Sjögren's syndrome. The action of sodium hyaluronate is due not only to its protective role of a mechanical type exerted on the epithelial cells as a result of its viscoelasticity, in situations of reduced tear production, but also to the positive effects of its particular biological function on corneal epithelial cells by stimulating their migration (Exp. Eye Res., 1991;53:753-758).

Vitamin E is the main antioxidant of the cell membranes, and is found in the human body in 4 forms consisting of α-tocopherol, β-tocopherol, γ-tocopherol and δ-tocopherol. Of these the α form is the most frequent in the retina and in plasma and is the one with the greatest antioxidant and free radical scavenging activity.

The use of inorganic elements is well known in the medical field, and a number of these are essential for the stability of the tear film (Contactologia, 1982; 4F:101-103).

In the medical field there is still a strongly perceived need for the availability of therapeutic agents or physiological supplement, useful for preventing or treating pathologies of the eyes due to ultraviolet radiation, which are not endowed with the drawbacks mentioned above.

It has now been found that a physiological supplement or medicament, in the form of eye drops or ointment, comprising as active ingredients (a) L-carnitine or a salt thereof; and (b) one or more solar filter or a salt or derivative thereof, in particular doses, is useful for preventing or treating pathologies of the eyes due to ultraviolet radiation.

What is meant by pharmaceutically acceptable salt of L-carnitine is any salt with an acid that does not give rise to toxic or side effects.

These acids are well known to pharmacologists and to experts in pharmacy. Non-limiting examples of such salts are: chloride, bromide, orotate, aspartate, acid aspartate, acid citrate, magnesium citrate, phosphate, acid phosphate, fumarate and acid fumarate, magnesium fumarate, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethanesulphonate, magnesium 2-amino- ethanesulphonate, methanesulphonate, choline tartrate, trichloroacetate, and trifluoroacetate.

What is meant by pharmaceutically acceptable salt of L-carnitine is also a salt approved by the FDA and listed in the publication Int. J. of Pharm. 33 (1986), 201-217, which is incorporated herein by way of a reference.

By solar filter according to the present invention it is meant a compound selected from the group comprising: 3-Hydroxykynurenine O-β-DL-glucoside or a derivative thereof selected from the group comprising: 3-Hydroxykynurenine O-β-D-glucoside; 3-Hydroxykynurenine O-β-L-glucoside; 3-hydroxykynurenine; 4-(2-amino-3-hydroxyphenyl)-4-oxobutanoic acid O-β-D-glucoside; 4-(2-amino-3-hydroxyphenyl)-4-oxobutanoic acid O-β-DL-glucoside; 4-(2-amino-3-hydroxyphenyl)-4-oxobutanoic acid O-β-L-glucoside; the glutathione adduct of 3-HKG; or an enantiomeric derivative thereof; or mixture thereof; or salts thereof.

It is therefore an object of the present invention a combination composition, in the form of eye drops, comprising the following components:
- L-carnitine from 0.01 to 10 g/100 mL, a preferred dose is from 0.1 to 4 g/100 mL, the most preferred dose is 2.00 g/100 mL;
- 3-HKG from 0.001 to 9 mg/100 mL, a preferred dose is from 0.1 to 5 mg/100 mL, the most preferred dose is 1 mg/100 mL;
- osmolality from 50 to 1200; preferred from 100 to 500, most preferred 250 mOsmols/kg;
   pH 6.3-7.8; preferred pH is 6.5-7.0; most preferred pH is 6.9;
for use for the prevention or treatment of diseases of the eye due to ultraviolet radiations.

It is a further object of the present invention the use of a combination composition comprising the following components:
- L-carnitine from 0.01 to 10 g/100 mL, a preferred dose is from 0.1 to 4 g/100 mL, the most preferred dose is 2.00 g/100 mL;
- 3-HKG from 0.001 to 9 mg/ 100 mL, a preferred dose is from 0.1 to 5 mg/100 mL, the most preferred dose is 1 mg/100 mL;
- osmolality from 50 to 1200; preferred from 100 to 500, most preferred 250 mOsmols/kg;
   pH 6.3-7.8; preferred pH is 6.5-7.0; most preferred pH is 6.9;
for the preparation of an ophthalmic composition in the form of eye drops, for the prevention or treatment of diseases of the eye due to ultraviolet radiations.

It is a further object of the present invention a method of prevention or treatment of diseases of the eye due to ultraviolet radiations, which comprise the administration into the eye of a patient in need thereof a suitable amount of a combination composition comprising as active ingredients the following components:
- L-carnitine from 0.01 to 10 g/100 mL, a preferred dose is from 0.1 to 4 g/100 mL, the most preferred dose is 2.00 g/100 mL;
- 3-HKG from 0.001 to 9 mg/100 mL, a preferred dose is from 0.1 to 5 mg/100 mL, the most preferred dose is 1 mg/100 mL;
- osmolality from 50 to 1200; preferred from 100 to 500, most preferred 250 mOsmols/kg;
pH 6.3-7.8; preferred pH is 6.5-7.0; most preferred pH is 6.9.

It is a further object of the present invention the use of a combination composition comprising the following components:
- L-carnitine from 0.01 to 10 g/100 mL, a preferred dose is from 0.1 to 4 g/100 mL, the most preferred dose is 2.00 g/100 mL;
- 3-HKG from 0.001 to 9 mg/100 mL, a preferred dose is from 0.1 to 5 mg/100 mL, the most preferred dose is 1 mg/100 mL;
- taurine from 0.005 to 50 g/100 mL, a preferred dose is from 0.05 to 5 g/100 mL, the most preferred dose is 0.50 g/100 mL;
- glycerol from 0.0025 to 25 g/100 mL, a preferred dose is from 0.025 to 2.5 g/100 mL, the most preferred dose is 0.25 g/100 mL;
- sodiun jaluronate from 0.002 to 20 g/100 mL, a preferred dose is from 0.02 to 2 g/100 mL, the most preferred dose is 0.20 g/100 mL;
- vitamine E acetate from 0.002 to 20 g/100 mL, a preferred dose is from 0.02 to 2 g/100 mL, the most preferred dose is 0.20 g/100 mL;
- resveratrol from 0.25 to 2500 mg/100 mL, a preferred dose is from 2.5 to 250 mg/100 mL, the most preferred dose is 25 mg/100;
- sodium from 0.3 to 3000 mg/L, a preferred dose is from 3.0 to 300 mg/L, the most preferred dose is 29 mg/L;
- potassium from 0.09 to 900 mg/L, a preferred dose is from 0.9 to 90 mg/L, the most preferred dose is 9 mg/L;
- zinc from 0.01 to 100 mg/L, a preferred dose is from 0.1 to 10 mg/L, the most preferred dose is 1.02 mg/L;
- manganese from 0.0005 to 5 mg/L, a preferred dose is from 0.005 to 0.5 mg/L, the most preferred dose is 0.05 mg/L;
- osmolality from 50 to 1200; preferred from 100 to 500, most preferred 250 mOsmols/kg;
   pH 6.3-7.8; preferred pH is 6.5-7.0; most preferred pH is 6.9;
for the preparation of an ophthalmic composition in the form of eye drops, for the prevention or treatment of diseases of the eye due to ultraviolet radiations.

The composition according to the present invention may be in the form of physiological supplement or medicament

The composition according to the present invention may be in the form of eye drops or ointment.

The compositions according to the present invention may further comprise antioxidants such as, for example, Borage oil; epithelialising and anti-angiogenic agents; humidifying agents; inorganic elements; vitamins, such as vitamin A, regulator of the cellular osmolarity; antibiotics; antiviral and antifungal agents, and/or one or more excipients and/or diluents physiologically acceptable.

The diseases of the eye due to ultraviolet radiation that can be prevented or treated according to the present invention can be selected from the group comprising: age-related cataract, non age-related cataract, pterygium, Macular Degeneration such as age related maculopathy (ARM); non age related maculopathy (nARM); age related macular degeneration (AMD), attinic photokeratitis and conjunctivitis.

The following non limiting examples further illustrate the invention.

### EXAMPLE 1

In order to evaluate the protective effect on the corneal epithelium against UV A/B radiation provided by the eye drops of the invention, the following experimental procedure was performed: 25 young male pigmented rabbits (60 days old) were subdivided into 5 equal groups, one of which did not undergo any treatment (negative control). In all the groups treated (groups 1-4) the right eye acted as a positive control. The following compositions were instilled respectively into the left eye every 2 hours for eight hours:
Group 1: Eye drops (complete composition):

| | |
|---|---|
| L-carnitine: | 2.00 g |
| 3-HKG: | 1 mg |
| Taurine: | 0.50 g |
| Sodium jaluronate: | 0.20 g |
| Glycerol: | 0.25 g |
| Resveratrol: | 25 mg |
| Vitamin E acetate: | 0.20 g |
| Zinc chloride (Zn): | 0.213 mg (0.102 mg) |
| Manganese chloride tetrahydrate (Mn): | 0.019 mg (0.005 mg) |
| Sodium chloride (Na): | 7.5 mg (2.9 mg) |
| Potassium chloride (K): | 1.72 mg (0.9 mg) |
| Cremophor RH 40 | 2.0 g |
| Microglicine /Gramcide III | 0.20 g |
| Methyl parahydrossibenzoate | 0.05 g |
| EDTA sodium | 0.05 g |
| NaOH 1 N | pH 6.8 - 7.5 |
| Demineralised steril water final volume | 100 mL |
| Colour: | Limpid yellowish |
| Viscosity | about 15 mPas |
| Osmolality | about 250 mOsmols/kg |

Group 2: Composition containing only L-carnitine 2%.
Group 3: Composition containing only 3-Hydroxykynurenine O-β-DL-glucoside 1 mg/100 mL.
Group 4: Composition containing taurine, glycerol, resveratrol, sodium jaluronate, vitamin E acetate, EDTA, cetrimide, sodium chloride, potassium chloride, zinc chloride and manganese chloride tetrahydrate.
Group 5: Untreated animals (Negative Control).

Then the animals were exposed for 2091 seconds to UV radiation using a xenon-mercury lamp, 5000 W, 290/360 nm, (Photochem. Photobiol. 1989; 49:175-180) after which the animals were killed and the eyeballs removed and placed in a fixative (4% paraformaldehyde). The cornea was then removed, included in paraffin and then evaluated for its morphological aspects and the cellular degeneration. The morphology was examined by means of hematoxylin-eosin staining whereas the cellular parameters were examined with vital exclusion stains and the TUNEL technique (analysis of DNA fragmentation, by means of terminal transferase, as a consequence of cellular stress) (Arch. Ophthalmol. 1987; 115:1031-1035).

The results obtained are reported in Table 1

**TABLE 1**

| | **EYE** | |
|---|---|---|
| | **LEFT** | **RIGHT** |
| | **Score** | **Score** |
| | **(arbitrary scale)** | **(arbitrary scale)** |
| Negative Control | 10 | 10 |
| | | |
| **Group 1:** complete composition | 10 | 1 |
| **Group 2:** L-carnitine 2% | 3 | 1 |
| | 4 | 1 |
| **Group 3:** 3-HKG 1 mg/100 mL | | |
| **Group 4:** taurine, sodium jaluronate, glycerol, resveratrol, vitamin E acetate, EDTA, cetrimide, sodium chloride, potassium chloride, zinc chloride and manganese chloride tetrahydrate. | 1 | 1 |

In all the right eyes (positive control) the epthelial cells degenerated and there was a clear increase in the cells with fragmented DNA. The morphology revealed a necrotic appearance of the tissue, albeit in the early stages, compared with the untreated animals.

On the other hand, in the left eyes, the analysis was different for the compositions indicated above.
Group 1: There were no significant variations compared with the eyes of the untreated animals (Negative Control, Group 5).
Group 2: The tissue appeared damaged, no damage to the DNA was seen.
Group 3: The tissue appeared damaged although this was not very apparent, no damage to the DNA was seen.
Group 4: The tissue appeared damaged with positive TUNEL reaction (DNA damaged).

The presence of the components of group 4 in group 2 or 3 respectively, did not modify the results obtained using L-carnitine alone (group 2) or 3-HKG alone (group 3); data not shown.

### EXAMPLE 2

In order to evaluate the protective effect of the eye drops being examined on the internal structures of the eye in subjects suffering from age-related maculopathy, the following experimental procedure was carried out.

24 Subjects between 49 and 74 years of age, irrespective of gender, received for 6 months the eye drops of Example 1 (complete composition) three times a day.

Subjects were subdivided into 4 equal groups and treated as reported in the following:
Group 1: Complete composition.
Group 2: Composition containing only L-carnitine 2%.
Group 3: Composition containing only 3-Hydroxykynurenine O-β-DL-glucoside 1 mg/100 mL.
Group 4: Composition containing taurine, glycerol, resveratrol, sodium jaluronate, vitamin E acetate, EDTA, cetrimide, sodium chloride, potassium chloride, zinc chloride and manganese chloride tetrahydrate.

The functional retinal status of the patients was evaluated using the focal electroretinogram (FERG) and the multifocal electroretinogram (mfERG) methods, before and after 6 months treatment. These two methods are useful for evaluating the electrical activity of the 30° retinal centres (Invest. Ophthalmol. Vis. Sci., 1986; 27 : 1123-1130; Eye, 2005; 19 : 431-441).

The results obtained revealed that there were no significant differences between the various groups in the amplitudes of the signal and in the phase.

In Group 1 there were no significant variations compared with the initial measurements.

In Group 2 a reduction of the in the amplitudes found with the FERG, whereas with the mfERG there was significant worsening.

In Group 3 a substantial stability was seen in the amplitudes found with the FERG, whereas with the mfERG there was a significant worsening.

In Group 4 there was a slight decrease in the amplitudes on FERG compared with the control, as was the case for area 52 of the mfERG carried out with 103 hexagons in the rings image. In spite of this, the value was significantly different compared with the values found initially.

**TABLE 2:**

| | **SCORE AFTER 6 MONTHS OF TREATMENT** |
|---|---|
| | **(ARBITRARY SCALE)** |
| **Group 1:** | 0 |
| **Group 2:** | - 6 |
| **Group 3:** | - 4 |
| **Group 4:** | - 8 |

| | |
|---|---|
| 0= no variation respect to time zero (beginning of the treatment). - 10= worsening of the symptoms respect to time zero. | |

L-carnitine is a known compound, the preparation process for which is described in US 4,254,053.

A method of preparation of 3-HKG is described in Anal Biochem. 2001 Dec 1;299(1):78-83. 3-Hydroxykynurenine O-P-DL-glucoside is a product sold by Sigma-Aldrich, catalogue 2006; product code n° H 1771.

The physiological supplement or medicament according to the present invention may be bought with or without medical prescription.

The physiological supplement or medicament according to the present invention are composed of active ingredients which are familiar to operators in the medical field and already in use in clinical practice, and their pharmacotoxicological profiles are known.

Their procurement therefore is very easy, inasmuch as these are products which have been on the market now for a long time and are of a grade suitable for human or animal administration.

In the following are reported non limiting examples of composition according to the present invention.

### Eye drops:

### Eye drops (complete composition):

| | |
|---|---|
| L-carnitine: | 2.00 g |
| 3-HKG: | 1 mg |
| Taurine: | 0.50 g |
| Sodium jaluronate: | 0.20 g |
| Glycerol (to obtain 250 mOsmols/kg): | 0.25 g |
| Resveratrol: | 25 mg |
| Vitamin E acetate: | 0.20 g |
| Zinc chloride (Zn): | 0.213 mg (0.102 mg) |
| Manganese chloride tetrahydrate (Mn): | 0.019 mg (0.005 mg) |
| Sodium chloride (Na) : | 7.5 mg (2.9 mg) |
| Potassium chloride (K): | 1.72 mg (0.9 mg) |
| Cremophor RH 40 | 2.0 g |
| Microglicine /Gramcide III | 0.20 g |
| Methyl parahydrossibenzoate | 0.05 g |
| EDTA sodium | 0.05 g |
| NaOH 1 N | pH 6.5-7.0 |
| Demineralised steril water | to 100 mL |
| Colour: | Limpid yellowish |
| Viscosity | about 15 mPas |
| Osmolality | about 250 mOsmols/kg. |

## Claims

1. Combination composition, in the form of eye drops, comprising the following components:
- L-carnitine, or a salt thereof, from 0.01 to 10 g/100 mL, a preferred dose is from 0.1 to 4 g/100 mL, the most preferred dose is 2.00 g/100 mL;
- 3-HKG, or a derivative thereof, from 0.001 to 9 mg/100 mL, a preferred dose is from 0.1 to 5 mg/100 mL, the most preferred dose is 1 mg/100 mL;
- osmolality from 50 to 1200; preferred from 100 to 500, most preferred 250 mOsmols/kg;
pH 6.3-7.8; preferred pH is 6.5-7.0; most preferred pH is 6.9;
for use for the prevention or treatment of diseases of the eye due to ultraviolet radiations.

2. Use of a combination composition comprising the following components:
- L-carnitine, or a salt thereof, from 0.01 to 10 g/100 mL, a preferred dose is from 0.1 to 4 g/100 mL, the most preferred dose is 2.00 g/100 mL;
- 3-HKG, or a derivative thereof, from 0.001 to 9 mg/100 mL, a preferred dose is from 0.1 to 5 mg/100 mL, the most preferred dose is 1 mg/100 mL;
- osmolality from 50 to 1200; preferred from 100 to 500, most preferred 250 mOsmols/kg;
pH 6.3-7.8; preferred pH is 6.5-7.0; most preferred pH is 6.9;
for the preparation of an ophthalmic composition in the form of eye drops for the prevention or treatment of diseases of the eye due to ultraviolet radiations.

3. Use of according to claims 1-2, further comprising: taurine; resveratrol; glycerol, sodium hyaluronate; one or more vitamins; and one or more inorganic elements.

4. Use of according to claim 3, in which the vitamin is Vitamin E.

5. Use of according to claim 3, in which the inorganic element is selected from the group consisting of: zinc; manganese; sodium or potassium.

6. Use of a combination composition comprising the following components:
- L-carnitine, or a salt thereof, from 0.01 to 10 g/100 mL, a preferred dose is from 0.1 to 4 g/100 mL, the most preferred dose is 2.00 g/100 mL;
- 3-HKG, or a derivative thereof, from 0.001 to 9 mg/100 mL, a preferred dose is from 0.1 to 5 mg/100 mL, the most preferred dose is 1 mg/100 mL;
- taurine from 0.005 to 50 g/100 mL, a preferred dose is from 0.05 to 5 g/100 mL, the most preferred dose is 0.50 g/100 mL;
- glycerol from 0.0025 to 25 g/100 mL, a preferred dose is from 0.025 to 2.5 g/100 mL, the most preferred dose is 0.25 g/100 mL;
- sodiun jaluronate from 0.002 to 20 g/100 mL, a preferred dose is from 0.02 to 2 g/100 mL, the most preferred dose is 0.20 g/100 mL;
- vitamine E acetate from 0.002 to 20 g/100 mL, a preferred dose is from 0.02 to 2 g/100 mL, the most preferred dose is 0.20 g/100 mL;
- resveratrol from 0.25 to 2500 mg/100 mL, a preferred dose is from 2.5 to 250 mg/100 mL, the most preferred dose is 25 mg/100;
- sodium from 0.3 to 3000 mg/L, a preferred dose is from 3.0 to 300 mg/L, the most preferred dose is 29 mg/L;
- potassium from 0.09 to 900 mg/L, a preferred dose is from 0.9 to 90 mg/L, the most preferred dose is 9 mg/L;
- zinc from 0.01 to 100 mg/L, a preferred dose is from 0.1 to 10 mg/L, the most preferred dose is 1.02 mg/L;
- manganese from 0.0005 to 5 mg/L, a preferred dose is from 0.005 to 0.5 mg/L, the most preferred dose is 0.05 mg/L;
- osmolality from 50 to 1200; preferred from 100 to 500, most preferred 250 mOsmols/kg;
pH 6.3-7.8; preferred pH is 6.5-7.0; most preferred pH is 6.9;
for the preparation of an ophthalmic composition in the form of eye drops for the prevention or treatment of diseases of the eye due to ultraviolet radiations.

7. Use of according to claims 1-6, in which the diseases of the eye due to ultraviolet radiation is selected from the group comprising:
age-related cataract, non age-related cataract, pterygium, macular degeneration such as age related maculopathy (ARM); non age related maculopathy (nARM); age related macular degeneration (AMD), attinic photokeratitis and conjunctivitis.

8. Use of according to claims 1-6, in which the composition is in the form of ointment.

9. Use according to claims 1-6, further comprising antioxidants, Borage oil; epithelialising and anti-angiogenic agents; humidifying agents; regulator of the cellular osmolarity; antibiotics; antiviral and antifungal agents, and/or one or more excipients and/or diluents physiologically acceptable.

10. Use of according to claims 1-6, in which the salt of L-carnitine is selected from the group consisting of chloride, bromide, orotate, aspartate, acid aspartate, acid citrate, magnesium citrate, phosphate, acid phosphate, fumarate and acid fumarate, magnesium fumarate, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethanesulphonate, magnesium 2-amino- ethanesulphonate, methanesulphonate, choline tartrate, trichloroacetate, and trifluoroacetate.

11. Use of according to claims 1-6, in which the derivative of 3-HKG is selected from the group consisting of: 3-Hydroxykynurenine O-P-D-glucoside; 3-Hydroxykynurenine O-β-L-glucoside; 3-hydroxykynurenine; 4-(2-amino-3-hydroxyphenyl)-4-oxobutanoic acid O-β-D-glucoside; 4-(2-amino-3-hydroxyphenyl)-4-oxobutanoic acid O-β-DL-glucoside; 4-(2-amino-3-hydroxyphenyl)-4-oxobutanoic acid O-β-L-glucoside; the glutathione adduct of 3-HKG; or an enantiomeric derivative thereof; or a salt thereof or mixture thereof.

12. Use of according to claims 1-6, in which the composition is in the form of physiological supplement or medicament.

13. Method of prevention or treatment of diseases of the eye due to ultraviolet radiations, which comprise the administration into the eye of a patient in need thereof a suitable amount of a combination composition comprising as active ingredients the following components:
- L-carnitine from 0.01 to 10 g/100 mL, a preferred dose is from 0.1 to 4 g/100 mL, the most preferred dose is 2.00 g/100 mL;
- 3-HKG from 0.001 to 9 mg/100 mL, a preferred dose is from 0.1 to 5 mg/100 mL, the most preferred dose is 1 mg/100 mL;
- osmolality from 50 to 1200; preferred from 100 to 500, most preferred 250 mOsmols/kg;
pH 6.3-7.8; preferred pH is 6.5-7.0; most preferred pH is 6.9.

14. Method of claim 13, in which the combination composition comprises the following components:
- L-carnitine, or a salt thereof, from 0.01 to 10 g/100 mL, a preferred dose is from 0.1 to 4 g/100 mL, the most preferred dose is 2.00 g/ 100 mL;
- 3-HKG, or a derivative thereof, from 0.001 to 9 mg/100 mL, a preferred dose is from 0.1 to 5 mg/100 mL, the most preferred dose is 1 mg/100 mL;
- taurine from 0.005 to 50 g/100 mL, a preferred dose is from 0.05 to 5 g/100 mL, the most preferred dose is 0.50 g/100 mL;
- glycerol from 0.0025 to 25 g/100 mL, a preferred dose is from 0.025 to 2.5 g/100 mL, the most preferred dose is 0.25 g/100 mL;
- sodiun jaluronate from 0.002 to 20 g/100 mL, a preferred dose is from 0.02 to 2 g/100 mL, the most preferred dose is 0.20 g/100 mL;
- vitamine E acetate from 0.002 to 20 g/100 mL, a preferred dose is from 0.02 to 2 g/100 mL, the most preferred dose is 0.20 g/100 mL;
- resveratrol from 0.25 to 2500 mg/100 mL, a preferred dose is from 2.5 to 250 mg/100 mL, the most preferred dose is 25 mg/100;
- sodium from 0.3 to 3000 mg/L, a preferred dose is from 3.0 to 300 mg/L, the most preferred dose is 29 mg/L;
- potassium from 0.09 to 900 mg/L, a preferred dose is from 0.9 to 90 mg/L, the most preferred dose is 9 mg/L;
- zinc from 0.01 to 100 mg/L, a preferred dose is from 0.1 to 10 mg/L, the most preferred dose is 1.02 mg/L;
- manganese from 0.0005 to 5 mg/L, a preferred dose is from 0.005 to 0.5 mg/L, the most preferred dose is 0.05 mg/L;
- osmolality from 50 to 1200; preferred from 100 to 500, most preferred 250 mOsmols/kg;
pH 6.3-7.8; preferred pH is 6.5-7.0; most preferred pH is 6.9.
